# FASCICULE DE BREVET EUROPEEN

(11) **EP 3 615 035 B1**
(45) Date de publication et mention de la délivrance du brevet: **09.06.2021**
(21) Numéro de dépôt: 18719583.9
(22) Date de dépôt: 27.04.2018
(51) Int. Cl.: A61K 31/575, A61K 36/28, A61P 21/00

(54) **UTILISATION DE 20-HYDROXYECDYSONE ET SES DÉRIVÉS DANS LE TRAITEMENT DES MYOPATHIES**
VERWENDUNG VON 20-HYDROXYECDYSON UND DERIVATEN DAVON ZUR BEHANDLUNG VON MYOPATHIEN
USE OF 20-HYDROXYECDYSONE AND THE DERIVATIVES THEREOF IN THE TREATMENT OF MYOPATHIES

(30) Priorité: 28.04.2017 FR 1753775; 31.08.2017 FR 1758071
(43) Date de publication de la demande: 04.03.2020
(73) Titulaire: Biophytis, 75001 Paris (FR); Sorbonne Université, 75006 Paris (FR)
(72) Inventeur: DILDA, Pierre, 75016 Paris (FR); LAFONT, René, 75016 Paris (FR); LATIL, Mathilde, 75019 Paris (FR); SEROVA, Maria, 91620 Nozay (FR); AGBULUT, Onnik, 92130 Issy-Les-Moulineaux (FR); VEILLET, Stanislas, 91600 Savigny sur Orge (FR)
(74) Mandataire: Ipside
(86) Numéro de dépôt international: PCT/EP2018/060975
(87) Numéro de publication internationale: WO 2018/197708

(56) Documents cités:
- FR-A1- 3 021 318
- MUTHITA HIRUNSAI ET AL: "Effect of 20-Hydroxyecdysone on Proteolytic Regulation in Skeletal Muscle Atrophy", IN VIVO: INTERNATIONAL JOURNAL OF EXPERIMENTAL AND CLINICAL PATHOPHYSIOLOGY AND DRUG RESEARCH, vol. 30, no. 6, 4 novembre 2016 (2016-11-04), pages 869-878, XP055457907, GR ISSN: 0258-851X, DOI: 10.21873/invivo.11007
- Anonymous: "Biophytis presents preliminary clinical data of SARA-PK, and new preclinical data of Sarconeos for treating sarcopenia", , 16 décembre 2016 (2016-12-16), pages 1-4, XP055430042, Extrait de l'Internet: URL:http://www.biophytis.com/wp-content/up loads/2015/05/PR-BIOPHYTIS-Posters-SCWD-16 1212_ENG-.pdf [extrait le 2017-11-29]
- Anonymous: "Biophytis", , 24 septembre 2015 (2015-09-24), pages 1-32, XP002778985, Extrait de l'Internet: URL:http://www.biophytis.com/wp-content/up loads/2015/09/INVEST-SECURITIES-BIOPHYTIS- REPORT-092215-EN.pdf [extrait le 2018-03-08]
- CHENG D M ET AL: "Continuous infusion of 20-hydroxyecdysone increased mass of triceps brachii in C57BL/6 mice", PHYTOTHERAPY RESEARCH, WILEY, UK, 1 avril 2013 (2013-04-01), pages 107-111, XP018507464, ISSN: 0951-418X, DOI: 10.1002/PTR.4679
- LI-JUN TAN ET AL: "Molecular genetic studies of gene identification for sarcopenia", HUMAN GENETICS, SPRINGER, BERLIN, DE, vol. 131, no. 1, 26 juin 2011 (2011-06-26) , pages 1-31, XP019992518, ISSN: 1432-1203, DOI: 10.1007/S00439-011-1040-7

## Description

### DOMAINE TECHNIQUE DE L'INVENTION

La présente invention concerne l'utilisation de 20-hydroxyecdysone (20E) naturelle purifiée ou des dérivés de synthèse pour le traitement des myopathies et notamment des dystrophies musculaires d'origine génétique incluant une mutation du gène de la dystrophine.

### ÉTAT DE LA TECHNIQUE

Les myopathies sont des maladies qui touchent directement le muscle. Il en existe différentes formes selon les manifestations et les mécanismes de l'atteinte musculaire. Deux catégories existent : les myopathies génétiques et les myopathies acquises.

Les myopathies génétiques se subdivisent en :
- dystrophies musculaires progressives (dystrophies musculaires de Duchenne et de Becker, dystrophies musculaires des ceintures et dystrophie facio-scapulo humérale), ou dystrophies congénitales,
- myopathies métaboliques telles que les glycogénoses et les lipidoses ainsi que les myopathies mitochondriales,
- dystrophies myotoniques telles que la myopathie de Steinert (DM1),
- myopathies congénitales de type central core, à bâtonnets, à multi mini core ou myopathie centronucléaire et myotubulaire,
Les myopathies acquises regroupent :
- les myopathies toxiques,
- les myopathies inflammatoires,
- les myopathies endocriniennes.

Les myopathies se manifestent par une faiblesse musculaire évolutive ou stable. Elles se caractérisent en général par une perte de masse musculaire (atrophie). Lors de l'évolution de la maladie, le tissu musculaire est progressivement remplacé par du tissu fibreux (fibrose).

Il existe plus de trente formes de dystrophies musculaires qui diffèrent notamment par la nature des muscles touchés. Elles apparaissent de manière plus ou moins précoce et touchent les muscles squelettiques de différentes parties du corps. Dans certains cas une atteinte progressive des muscles respiratoires et cardiaque réduit l'espérance de vie des patients.

Plusieurs dizaines de gènes différents sont impliqués dans les dystrophies musculaires. Le plus souvent, ce sont des gènes responsables de la synthèse de protéines situées dans la membrane des cellules musculaires ou liées à celle-ci et indispensables au maintien de l'architecture et à la fonction musculaire. A titre d'exemple :
- la dystrophine est impliquée dans la dystrophie musculaire de Duchenne (DMD) et la dystrophie musculaire de Becker (BMD),
- la calpaïne, la dysferline les sarcoglycanes sont impliqués dans les dystrophies musculaires des ceintures,
- la mérosine, l'alpha-dystroglycane ou la sélénoprotéine N sont impliquées dans le cas des dystrophies musculaires congénitales (DMC).

La DMD est la plus fréquente des dystrophies musculaires. Elle touche 1 garçon sur 3500 et résulte de mutations affectant le gène de la dystrophine, localisé sur le chromosome X. Une forme moins sévère, la BMD, implique également le gène de la dystrophine et touche 1 garçon sur 18000. La DMD est une affection génétique grave qui touche toute la musculature. Dans cette maladie, la fragilité des fibres musculaires aboutit à leur destruction entraînant une nécrose du tissu musculaire. Lorsque les mécanismes de régénération sont dépassés, la dégénérescence l'emporte entraînant une perte de force musculaire et une intolérance à l'effort (Barnabei et al. 2011). Les fibres musculaires sont alors remplacées par du tissu conjonctif (fibrose). Une faiblesse musculaire gagne progressivement les membres inférieurs des enfants âgés de plus de 3 ans. La maladie se développe par la suite dans les muscles du dos, les membres supérieurs et enfin les muscles respiratoires.

Il n'existe actuellement aucun traitement curatif à la DMD et la BMD, mais les traitements palliatifs, orthopédiques et respiratoires, améliorent la qualité de vie et le pronostic vital. La prise en charge des patients repose actuellement sur l'optimisation de leurs capacités musculaires ainsi que sur la prévention et le traitement des complications cardiaques et respiratoires. L'utilisation de corticoïdes permet de prolonger la période de marche de deux ans en moyenne. Toutefois, certains enfants ne répondent pas à ce traitement qui de plus est responsable d'effets indésirables, notamment une fragilisation osseuse importante. Une protection cardiaque partielle est obtenue grâce à une l'association d'inhibiteurs de l'enzyme de conversion et de bêta-bloquants.

De nouveaux traitements sont au stade du développement clinique. Le saut d'exon consiste à forcer la cellule à produire une version de la dystrophine plus courte que la protéine normale mais cependant fonctionnelle. Une autre approche du même type consiste à passer outre une mutation qui interrompt prématurément la synthèse de la dystrophine. Toutefois, ce type de thérapie ne s'adresse qu'à un petit nombre de patients, et ce en fonction de la nature exacte des mutations à l'origine de leur maladie. Enfin, la thérapie génique, qui offre la possibilité de synthétiser des versions courtes de dystrophine (mini-dystrophines ou micro-dystrophines) chez les patients, se trouve confrontée un problème majeur de réponse immunitaire contre ces protéines considérées comme étrangères par l'organisme.

Outre une diminution irréversible de la force musculaire appendiculaire et l'apparition d'une intolérance à l'effort, une des complications majeures de la maladie est l'installation d'une fibrose qui touche notamment le cœur conduisant à une insuffisance cardiaque (accompagnée d'une hypertrophie dilatée). Ces atteintes engagent le pronostic vital des patients. L'installation de la fibrose est par conséquent une évolution irréversible qu'il convient de contrecarrer afin de conserver la fonction musculaire. Dans ce sens, les approches thérapeutiques doivent concentrer leurs efforts sur :
- le maintien de la tolérance à l'effort
- le maintien de la force musculaire
- et sur la prévention de l'apparition de la fibrose.

Les phytoecdysones représentent une importante famille de stérols polyhydroxylés. Ces molécules sont produites pas diverses espèces de plantes (fougères, gymnospermes, angiospermes) et participent à la défense de ces plantes contre les ravageurs.

Le brevet FR3 021318 divulgue que les phytoecdysones, et plus particulièrement la 20-hydroxyecdysone (20E), ont fait l'objet de nombreuses études pharmacologiques. Ces études ont mis en avant les propriétés antidiabétiques et anabolisantes de cette molécule. Ses effets stimulants sur les synthèses protéiques dans les muscles sont observés chez le rat *in vivo* (Syrov et al., 2000; Tóth et al., 2008; Lawrence et al., 2012) et sur des myotubes murins C2C12 *in vitro* (Gorelick-Feldman et al., 2008). Il s'agit d'un effet au niveau de la traduction, qui implique la phosphorylation de la protéine ribosomale p70S6K, à l'issue d'une cascade où intervient la protéine kinase Akt/PkB, une voie également utilisée par l'IGF-1 pour stimuler la protéosynthèse.

Certains des effets décrits ci-dessus dans des modèles animaux ont été retrouvés dans des études cliniques, encore peu nombreuses. Ainsi, la 20-hydroxyecdysone augmente la masse musculaire chez de jeunes sportifs (Simakin et al., 1988).

Enfin le brevet français FR3021318 décrit l'utilisation de 20-hydroxyecdysone et ses dérivés, pour le traitement et la prévention de la sarcopénie et de l'obésité sarcopénique (Lafont et al. 2017).

### OBJET DE L'INVENTION

Les inventeurs ont découvert que la 20-hydroxyecdysone et ses dérivés améliorent de manière significative les performances physiques in toto ainsi que la force musculaire in situ de mammifères atteints par une myopathie. Les performances physiques in toto et la force musculaire in situ sont déterminées respectivement par la mesure de distance maximale parcourue et par la force maximale isométrique absolue du muscle tibial antérieur. Ces effets permettent d'améliorer la mobilité chez les mammifères atteints de myopathie et notamment de dystrophies musculaires.

La présente invention vise la 20-hydroxyecdysone et ses dérivés destinés à être utilisés dans le traitement d'une myopathie résultant d'une altération génétique incluant une mutation du gène de la dystrophine.

Dans la suite de la description, on entend par 20-hydroxyecdysone et ses dérivés, la 20-hydroxyecdysone, ses dérivés, des extraits de végétaux riches en 20-hydroxyecdysone et ses dérivés, et des compositions comportant à titre d'agent actif la 20-hydroxyecdysone, ses dérivés et/ou des extraits de végétaux riches en 20-hydroxyecdysone et ses dérivés.

Les dérivés de 20-hydroxyecdysone sont obtenus par hémisynthèse.

La 20-hydroxyecdysone et ses dérivés sont avantageusement purifiés au grade pharmaceutique.

Plus particulièrement, l'invention concerne la 20-hydroxyecdysone et ses dérivés, destinés à être utilisés dans le traitement d'affections résultant d'une altération de la fonction musculaire provoquée soit par une myopathie génétique.

La fonction musculaire est celle du muscle strié squelettique ou du myocarde.

Plus particulièrement, l'altération de la fonction musculaire est une hypertrophie du myocarde.

Encore plus particulièrement l'invention concerne la 20-hydroxyecdysone et ses dérivés, destinés à être utilisés dans le traitement de certaines myopathies dans lesquelles la fonction musculaire est au moins en partie altérée par l'installation progressive d'une fibrose.

L'invention concerne la 20-hydroxyecdysone et ses dérivés, destinés à être utilisés dans le traitement d'une myopathie résultant d'une altération génétique, notamment du gène de la dystrophine.

Par altération génétique on entend une mutation, une insertion de nucléotide(s) ou une délétion de nucléotide(s).

L'invention concerne la 20-hydroxyecdysone et ses dérivés, destinés à être utilisés dans le traitement par exemple de la dystrophie musculaire de Duchenne (DMD) et/ou la dystrophie musculaire de Becker (BMD).

Selon l'invention, la 20-hydroxyecdysone et ses dérivés, sont destinés à être utilisés dans le traitement de toute myopathie résultant d'une mutation du gène de la dystrophine.

Selon une caractéristique, la 20-hydroxyecdysone est un composé de formule (I):

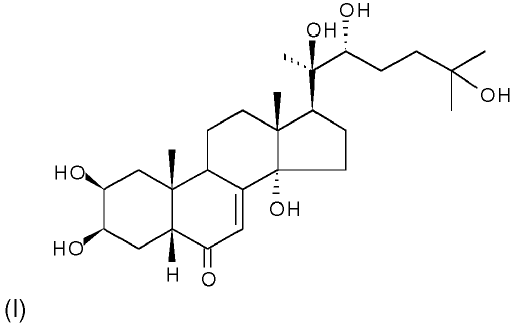

De manière avantageuse, le composé de formule (I) est purifié au grade pharmaceutique.

Tout particulièrement, la 20-hydroxyecdysone est un extrait de plante ou d'une partie de plante, ladite plante étant choisie parmi les végétaux contenant au moins 0,5% de 20-hydroxyecdysone de formule (I) en poids sec dudit végétal, ledit extrait comportant au moins 95%, et de préférence au moins 97%, de 20-hydroxyecdysone de formule (I).

On parle de purification au grade pharmaceutique.

L'extrait est appelé par la suite BIO101. Il comporte de façon remarquable entre 0 et 0,05 %, en poids sec de l'extrait, d'impuretés, comme des composés mineurs, susceptibles d'affecter l'innocuité, la disponibilité ou l'efficacité d'une application pharmaceutique dudit extrait.

Selon une caractéristique de l'invention, les impuretés sont des composés à 19 ou 21 atomes de carbone, comme la Rubrostérone, la Dihydrorubrostérone ou la Poststérone.

La plante à partir de laquelle est produit BIO101 est avantageusement choisie parmi *Stemmacantha carthamoides* (aussi appelée *Leuzea carthamoides*), *Cyanotis arachnoidea* et *Cyanotis vaga.*

L'invention s'intéresse tout spécialement à l'utilisation d'un extrait de racines de *Stemmacantha carthamoides* comportant au moins 95%, et de préférence au moins 97%, de 20-hydroxyecdysone de formule (I).

Dans cet extrait, la 20-hydroxyecdysone est purifiée au grade pharmaceutique.

Plus particulièrement, l'extrait est administré à raison de 3 à 15 mg/kg*jour.

Tout particulièrement, l'extrait est administré à raison de 200 à 1000 mg/jour, en une ou plusieurs prises, chez un patient adulte, et une dose de 70 à 350 mg/jour, en une ou plusieurs prises, chez l'enfant.

En outre l'invention concerne une composition comportant à titre d'agent actif BIO101.

La composition contient de préférence entre 200 et 1000 mg d'agent actif (BIO101).

Selon une autre caractéristique, un dérivé de la 20-hydroxyecdysone est un composé de formule générale (II) : 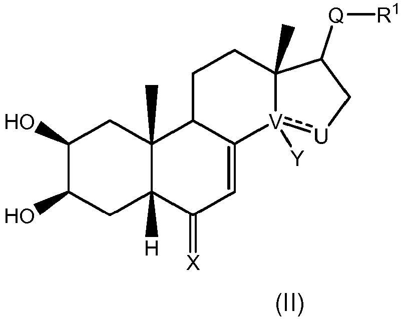 dans laquelle :
**V-U** est une liaison simple carbone-carbone et **Y** est un groupement hydroxyle ou un hydrogène, ou **V-U** est une liaison éthylénique C=C ;
**X** est un oxygène,
**Q** est un groupement carbonyle ;
**R¹** est choisi parmi : un groupement (C₁-C₆)**W**(C₁-C₆) ; un groupement (C₁-C₆)**W**(C₁-C₆)**W**(C₁-C₆) ; un groupement (C₁-C₆)**W**(C₁-C₆)CO₂(C₁-C₆); un groupement (C₁-C₆)**A**, **A** représentant un hétérocycle éventuellement substitué par un groupement de type OH, OMe, (C₁-C₆), N(C₁-C₆), CO₂(C₁-C₆) ; un groupement CH₂Br;
**W** étant un hétéroatome choisi parmi N, O et S, de préférence O et encore plus préférentiellement S.

Plus particulièrement, dans la formule (II) :
**Y** est un groupement hydroxyle ;
**R¹** est choisi parmi : un groupement (C₁-C₆)**W**(C₁-C₆) ; un groupement (C₁-C₆)**W**(C₁-C₆)**W**(C₁-C₆) ; un groupement (C₁-C₆)**W**(C₁-C₆)CO₂(C₁-C₆); un groupement (C₁-Cₑ)**A**, **A** représentant un hétérocycle éventuellement substitué par un groupement de type OH, OMe, (C₁-C₆), N(C₁-C₆), CO₂(C₁-C₆);
**W** étant un hétéroatome choisi parmi N, O et S, de préférence O et de préférence encore S.

En particulier, les composés de formule (II) sont choisis parmi :
- **n° 1 :** (2S,3R,5R,10R,13R,14S,17S)-2,3,14-trihydroxy-10,13-diméthyl-17-(2-morpholinoacétyl)-2,3,4,5,9,11,12,15,16,17-décahydro-1H-cyclopenta[a]phénanthrèn-6-one,
- **n° 2 :** (2S,3R,5R,10R,13R,14S,17S)-2,3,14-trihydroxy-17-[2-(3-hydroxypyrrolidin-1-yl)acétyl]-10,13-diméthyl-2,3,4,5,9,11,12,15,16,17-décahydro-1H-cyclopenta[a]phénanthrèn-6-one;
- **n°3 :** (2S,3R,5R,10R,13R,14S,17S)-2,3,14-trihydroxy-17-[2-(4-hydroxy-1-pipéridyl)acétyl]-10,13-diméthyl-2,3,4,5,9,11,12,15,16,17-décahydro-1H-cyclopenta[a]phénanthrèn-6-one;
- **n°4 :** (2S,3R,5R,10R,13R,14S,17S)-2,3,14-trihydroxy-17-[2-[4-(2-hydroxyéthyl)-1-pipéridyl]acétyl]-10,13-diméthyl-2,3,4,5,9,11,12,15,16,17-décahydro-1H-cyclopenta[a]phénanthrèn-6-one;
- **n°5 :** (2S,3R,5R,10R,13R,14S,17S)-17-[2-(3-diméthylaminopropyl(méthyl)amino)acétyl]-2,3,14-trihydroxy-10,13-diméthyl-2,3,4,5,9,11,12,15,16,17-décahydro-1H-cyclopenta[a]phénanthrèn-6-one;
- **n°6 :** 2-[2-oxo-2-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]sulfanylacétate d'éthyle;
- **n°7 :** (2S,3R,5R,10R,13R,14S,17S)-17-(2-éthylsulfanylacétyl)-2,3,14-trihydroxy-10,13-diméthyl-2,3,4,5,9,11,12,15,16,17-décahydro-1H-cyclopenta[a]phénanthrèn-6-one;
- **n°8 :** (2S,3R,5R,10R,13R,14S,17S)-2,3,14-trihydroxy-17-[2-(2-hydroxyéthylsulfanyl)acétyl]-10,13-diméthyl-2,3,4,5,9,11,12,15,16,17-décahydro-1H-cyclopenta[a]phénanthrèn-6-one.

Tour particulièrement, la phytoecdysone est un composé de formule (III) :

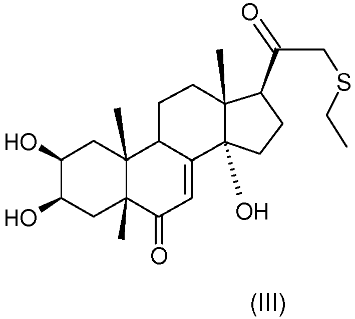

Ce composé est appelé par la suite BIO103.

### BRÈVE DESCRIPTION DES FIGURES

D'autres avantages, buts et caractéristiques particulières de la présente invention ressortiront de la description non limitative qui suit d'au moins un mode de réalisation particulier des dispositifs objets de la présente invention, en regard des dessins annexés, dans lesquels :
- La figure 1A est un diagramme représentatif de la tolérance à l'effort des groupes de souris de fond génétique C57BL10 : saines (C57) et mdx (mutées sur le gène de la dystrophine) non traitées,
- La figure 1B est un diagramme représentatif de la tolérance à l'effort des groupes de souris : mdx non traitées, mdx traitées par BIO101 et mdx traitées par BIO103 après deux mois de traitement,
- La figure 2A est un diagramme représentatif de la force isométrique maximale absolue du muscle tibial antérieur des groupes de souris de fond génétique C57BL10 : saines (C57) et mdx non traitées,
- La figure 2B est un diagramme représentatif de la force isométrique maximale absolue du muscle tibial antérieur des groupes de souris : mdx non traitées, mdx traitées par BIO101 et mdx traitées par BIO103 après deux mois de traitement,
- Les figures 3A et 3B sont des diagrammes représentatifs de l'expression génique (ARNm) des marqueurs de fibrose CTGF (facteur de croissance de tissu conjonctif) et Colla1 (collagène 1) du coeur de différents groupes de souris de fond génétique C57BL10 : saines (C57), mdx non traitées, mdx traitées par BIO101 et mdx traitées par BIO103, après deux mois de traitement,
- Les figures 3C et 3D sont des diagrammes représentatifs de l'expression génique (ARNm) des marqueurs d'hypertrophie myh7 (chaîne lourde bêta de la myosine) et BMP4 (protéine morphogénétique osseuse 4) du coeur de différents groupes de souris de fond génétique C57BL10 : saines (C57), mdx non traitées, mdx traitées par BIO101 et mdx traitées par BIO103, après deux mois de traitement,
- La figure 4A est une image représentative de coupe histologique de muscle tibial antérieur de souris de fond génétique C57BL10 saines (C57), coloré à l'hématoxyline éosine (HE),
- La figure 4B est une image représentative de coupe histologique de muscle tibial antérieur de souris de fond génétique C57BL10 mdx non traitées (mdx), coloré à l'hématoxyline éosine (HE),
- La figure 4C est une image représentative de coupe histologique de muscle tibial antérieur de souris de fond génétique C57BL10 mdx traitées par BIO101 (mdx+BIO101), coloré à l'hématoxyline éosine (HE)
- La figure 4D est une image représentative de coupe histologique de muscle tibial antérieur de souris de fond génétique C57BL10 mdx traitées par BIO103 (mdx+BIO103), coloré à l'hématoxyline éosine (HE),
- Les figures 5A et 5B sont des coupes histologiques représentatives de muscle tibial antérieur colorées au rouge sirius (RS) respectivement des groupes de souris de fond génétique C57BL10 saines (C57) et mdx non traitées (mdx),
- La figure 5C est un diagramme représentatif de la quantification des zones de fibrose des groupes de souris de fond génétique C57BL10 saines (C57) et mdx non traitées (mdx),
- Les figures 5D et 5E sont des coupes histologiques représentatives de muscle tibial antérieur colorées au rouge sirius (RS) des groupes de souris mdx traitées par BIO101 (mdx+BIO101) et mdx traitées par BIO103 (mdx+BIO103),
- La figure 5F est un diagramme représentatif de la quantification des zones de fibrose des groupes de souris mdx traitées par BIO101 (mdx BIO101) et mdx traitées par BIO103 (mdx BIO103),
- La figure 6A présente l'expression protéique, détectée par western blot, du marqueur de fibrose collagène 1 (Col1a1) du muscle gastrocnémien des groupes de souris de fond génétique C57BL10 : saines (C57), mdx non traitées (mdx),
- La figure 6B présente l'expression protéique, détectée par western blot, du même marqueur de fibrose (Col1a1) du muscle gastrocnémien des groupes de souris de fond génétique C57BL10 : mdx non traitées (mdx), mdx traitées par BIO101 et mdx traitées par BIO103, après deux mois de traitement,
- la figure 7A est un diagramme représentatif de l'indice de fusion de myoblastes en myotubes. Les myoblastes humains utilisés proviennent d'un patient atteint de DMD. Les myoblastes en cours de différenciation ont été traités par BIO101 ou le véhicule pendant 3 jours,
- la figure 7B est un diagramme représentatif du nombre de noyaux par myotube. Les myoblastes humains utilisés proviennent d'un patient atteint de DMD. Les myoblastes en cours de différenciation ont été traités par BIO101 ou le véhicule pendant 3 jours,
- la figure 7C est un diagramme représentatif du diamètre des myotubes. Les myoblastes humains utilisés proviennent d'un patient atteint de DMD. Les myoblastes en cours de différenciation ont été traités par BIO101 ou le véhicule pendant 3 jours,
- les figures 8A et 8B présentent l'expression protéique, détectée par western blot, des formes phosphorylées des protéines AKT et ERK1/2 dans des myoblastes humains provenant d'un patient atteint de DMD. Les myoblastes en cours de différenciation ont été traités par BIO101 pendant une durée comprise entre 10 min et 24 heures.
- les figures 9A et 9B présentent respectivement la respiration basale et maximale de myoblastes humains provenant d'un patient atteint de DMD. Les myoblastes en cours de différenciation ont été traités par le véhicule ou BIO101 à différentes doses pendant trois jours. La respiration des cellules est déterminée par la mesure de la vitesse de consommation d'oxygène.
- la figure 10A est un diagramme représentatif de la quantification des vaisseaux sanguins entourant chaque fibre musculaire des groupes de souris de fond génétique C57BL10 saines (C57) et mdx non traitées,
- la figure 10B est un diagramme représentatif de la quantification des vaisseaux sanguins entourant chaque fibre musculaire des groupes de souris mdx traitées par le véhicule (mdx), BIO101 (mdx BIO101) et mdx traitées par BIO103 (mdx BIO103).

### DESCRIPTION DÉTAILLÉE DE MODES DE RÉALISATION DE L'INVENTION

La présente description est donnée à titre non limitatif.

### 1. Procédé de purification de BIO101

BIO101 est préparé à partir d'une préparation de 20-hydroxyecdysone pure de l'ordre de 90%, selon les étapes suivantes :
a) Dissolution à chaud de 20-hydroxyecdysone pure de l'ordre de 90% dans du méthanol, filtration et concentration partielle,
b) Ajout de 3 volumes d'acétone,
c) Refroidissement à une température comprise entre 0 et 5°C, sous agitation,
d) Filtration du précipité obtenu,
e) Rinçages successifs avec de l'acétone et de l'eau, et
f) Séchage.

Cette purification met en jeu un processus de recristallisation approprié à cette molécule et apte à être réalisé à l'échelle industrielle.

La filtration de l'étape a) est réalisée grâce à un filtre à particules de 0,2 µm (micromètres).

La concentration partielle de l'étape a) est avantageusement effectuée par distillation sous vide, à une température de l'ordre de 50°C, en présence de MeOH.

L'étape f) de séchage est effectuée sous vide à une température de l'ordre de 50°C.

### 2. Procédé de synthèse de BIO103

BIO103 est obtenu par hemi-synthèse à partir de à partir de 20-hydroxyecdysone puis purification au grade pharmaceutique selon le procédé de préparation suivant :

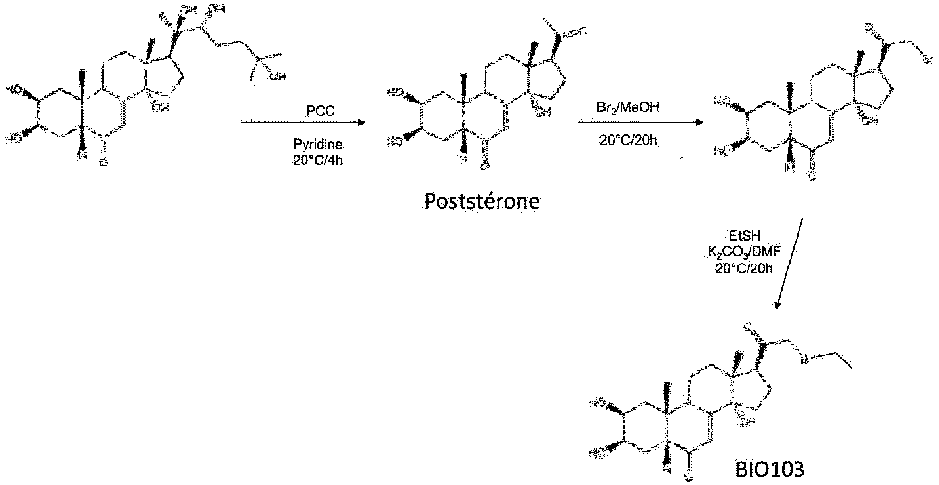

Schéma de synthèse de BIO103 en 3 étapes :
1) coupure oxydante de la chaîne latérale de la 20-hydroxyecdysone entre les carbones C20 et C22 pour obtenir la poststérone (protocole connu de l'homme du métier),
2) introduction d'un atome de brome en position C21,
3) réaction du dérivé bromé avec de l'éthane-thiol.

### 3. Activité biologique de BIO101 et BIO103

Si les effets anabolisants de la 20E présente dans des préparations commerciales ont déjà été démontrés chez des animaux jeunes, on ne connait pas l'effet de la 20E dans un contexte de mammifère atteint de myopathie dans laquelle l'installation progressive d'une fibrose contribue à l'altération de la fonction musculaire. Le modèle animal de la myopathie de Duchenne employant des souris mdx, de fond génétique C57BL10, qui présentent une mutation sur le gène de la dystrophine, a été mis en œuvre (Bulfield et al. 1984 ; Sicinski et al. 1989).

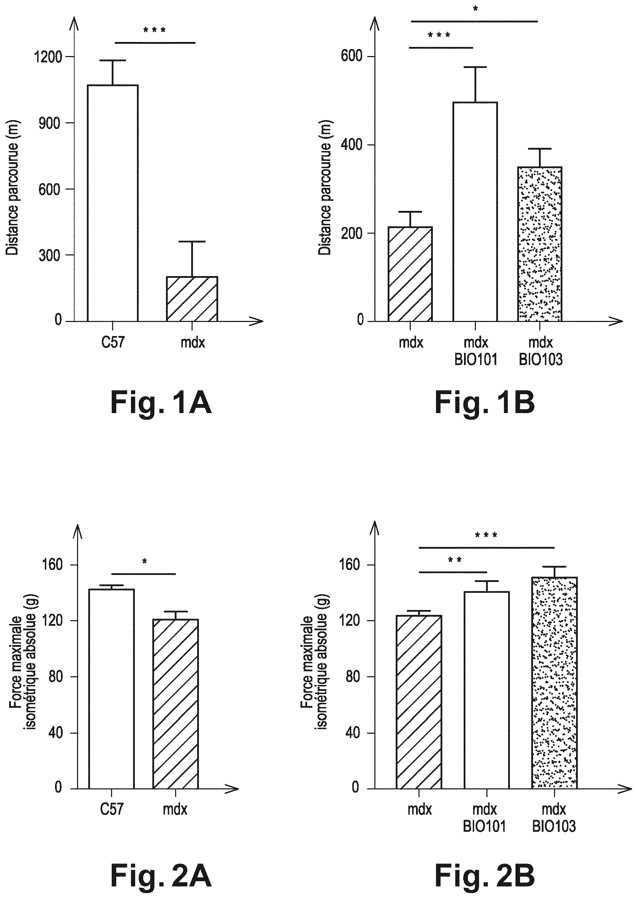

Des souris mâles de fond génétique C57BL10 (souris sauvages dénommées «C57» dans les figures) et C57BL10 mdx (modèle murin de la dystrophie musculaire de Duchenne, dénommées «mdx» dans les figures) de douze semaines, produites chez Charles Rivers, ont été utilisées. Deux groupes des souris mdx ont été exposés par voie orale de manière chronique soit à BIO101 (nombre de souris n=9) soit à BIO103 (nombre de souris n=9) à la dose de 50 mg/kg*jour. Un groupe de souris C57 (nombre de souris n=5) et un groupe des souris mdx (nombre de souris n=15) ont reçu le véhicule, soit aucun traitement. Les animaux de tous les groupes ont été testés pour leur capacité fonctionnelle (test de tolérance à l'effort) après deux mois de traitement. Le test de tolérance à l'effort consiste à mesurer la distance maximale parcourue (activité *in toto;* Figures 1A et 1B). En outre, des mesures de la force maximale isométrique absolue du muscle tibial antérieur (activité *in situ* ; Figures 2A et 2B) ont été effectuées après deux mois de traitement.

Le traitement par voie orale sur deux mois consiste au gavage pendant cinq jours par semaine et dans l'eau de boisson deux jours par semaine.

### Test de tolérance à l'effort (étude fonctionnelle in toto : figures 1A et 1B)

Le test de tolérance à l'effort est un exercice forcé sur tapis roulant motorisé. C'est une méthode non invasive pour évaluer la fonction du muscle squelettique in toto. C'est la méthode de référence dans le domaine (Ferry et al. 1992, 1993 ; Hadj-Said et al. 2012).

La durée d'acclimatation des animaux est au moins de 48h avant la séance durant laquelle est mesurée la distance maximale de course. Durant le test, la souris est placée dans un couloir de course sur un tapis roulant motorisé qui permet de faire un exercice contrôlé par l'expérimentateur dans son intensité et sa durée. Le tapis roulant se termine par une grille horizontale qui administre un choc électrique (0,4 mA) si l'animal est en contact plus d'une seconde avec la grille.

La séance de course débute par une période d'échauffement de 2 minutes pendant laquelle la vitesse est amenée de 0 à 20 cm/s. Par la suite, la vitesse de course est augmentée de 5 cm/s toutes les 10 minutes jusqu'à la limite des capacités maximales de la souris. Lorsqu'elle subit 5 chocs en moins de 10 secondes, le test s'arrête. La distance parcourue est notée.

De manière attendue, il est constaté que les animaux qui présentent une mutation sur le gène de la dystrophine (mdx) courent significativement moins (-80,4%, p<0.001, test Mann Whitney) que les animaux sains (C57) (Figure 1A). Après deux mois d'exposition quotidienne (Figure 1B), les animaux mdx qui ont reçu BIO101 ou BIO103 courent quant à eux significativement plus que les animaux mdx qui ont reçu le véhicule. BIO101 et BIO103 améliorent significativement (respectivement p<0,001 et p<0,05, test t non-apparié) la distance parcourue respectivement de +136% et +67% par rapport à celle des animaux mdx non traités (véhicule). De manière importante, nous démontrons ici que le traitement des animaux mdx par BIO101 ou BIO103 compense en partie la perte fonctionnelle significative observée chez les animaux mutés sur le gène de la dystrophine (mdx). Les performances physiques globales (activité *in toto*) des animaux atteints de myopathie sont très nettement améliorées par BIO101 (2,4 fois) et BIO103 (1,7 fois).

Cette étude démontre qu'un traitement par BIO101 ou BIO103, se traduit par une amélioration fonctionnelle caractérisée par une amélioration significative de la tolérance à l'effort dans un modèle animal de myopathie (Figures 1A et 1B).

### Force maximale isométrique absolue du muscle tibial antérieur (étude fonctionnelle in situ : figure 2)

Une évaluation de la contractilité in situ du muscle tibial antérieur est réalisée en fin de protocole, soit après deux mois de traitement.

Le jour du sacrifice, la souris est anesthésiée avec une injection intrapéritonéale de pentobarbital (55 mg/kg, 0,1 mL/10 g de poids corporel) avant la mesure de force in situ du muscle tibial antérieur (TA). La peau du dessus de la patte est incisée, ce qui laisse apparaître le tendon qui est sectionné à son extrémité distale. Le tendon distal du TA est attaché au levier du servomoteur (305B Dual-Mode Lever, Aurora Scientific). La peau sur la face latérale de la cuisse est incisée, ce qui laisse apparaître le nerf sciatique, entre 2 groupes musculaires. Le nerf sciatique est stimulé avec une électrode bipolaire (pulse supra-maximal d'onde carrée de 10V, 0,1 ms). La force est mesurée pendant des contractions en réponse à la stimulation électrique (fréquence de 75-150 Hz, durée de 500 ms). La température de la souris est maintenue à 37°C à l'aide de lampe radiante. La force maximale tétanique isométrique absolue est mesurée.

De manière attendue, il est constaté que les animaux mdx ont une force de contraction maximale isométrique absolue significativement moins importante (p<0.05, test t non-apparié) que celle des animaux sains C57 (Figure 2A). Après deux mois de traitement quotidien, une augmentation significative de la force maximale isométrique absolue du muscle tibial antérieur est observée chez les animaux mdx traités par BIO101 (+15,3%, p<0,05, test t non apparié) et par BIO103 (+22,5%, p<0.001, test t non apparié) à la dose de 50 mg/kg*jour par rapport aux animaux mdx ayant reçu le véhicule (Figure 2B).

Cette étude démontre qu'un traitement par BIO101 ou BIO103, se traduit par une amélioration fonctionnelle caractérisée par une augmentation significative de la force maximale isométrique absolue du muscle tibial antérieur dans un modèle animal de myopathie (Figures 2A et 2B).

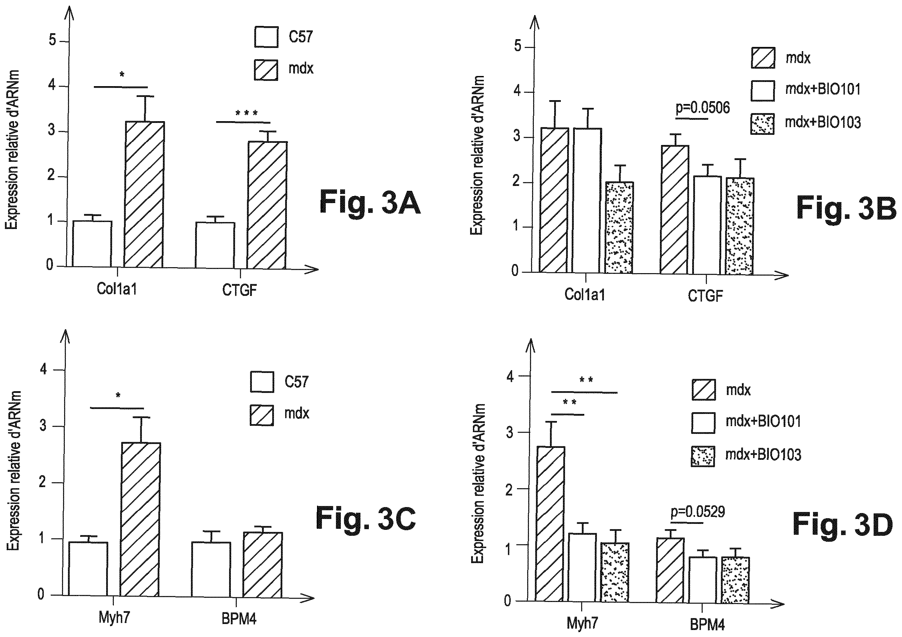

### Fibrose du myocarde et hypertrophie cardiaque (étude moléculaire : figures 3A, 3B, 3C, 3D)

Une évaluation de marqueurs de fibrose et d'hypertyrophie cardiaque est réalisée en fin de protocole, soit après deux mois de traitement. Le jour du sacrifice, les souris sont euthanasiées par décapitation et le cœur est prélevé puis congelé immédiatement dans l'azote liquide. L'ARN total est extrait avec du TRIzol® lysis Reagent (Life technologies) et un homogénéisateur tissulaire (Bio-Gen PRO200). Les ARN extraits sont quantifiés par spectrophotométrie puis leur qualité est vérifiée et validée par le kit Experion RNA StdSens Analysis Kit (Bio-Rad). L'ADNc est ensuite synthétisé avec le kit RevertAid First Strand cDNA Synthesis Kit (Thermo Fisher Scientific). Enfin, l'analyse semi-quantitative en PCR a été réalisée en utilisant SYBR® Green (Roche), un intercalant de l'ADN et un appareil LightCycler® 480 Real Time PCR (Roche) sur plaques 384 puits. Les marqueurs moléculaires mesurés sont les suivants :
- CTGF et collagène I : le facteur de croissance de tissu conjonctif (CTGF) est associé à la cicatrisation et à de nombreux processus fibrotiques pathologiques dont la DMD (Brigstock 2010; Song Y. 2017). Une augmentation de l'expression génique de CTGF est associée à la fibrose cardiaque chez la souris mdx (Au 2011). CTGF stimule la synthèse de collagène (dont le collagène I) dans les fibroblastes cardiaques et contribue à la fibrose du myocarde (Wang 2010),
- myh7 : une augmentation de l'expression d'ARNm de la chaine lourde de bêta myosine (myh7) est liée à une hypertrophie cardiaque. Cette isoforme embryonnaire s'exprime dans le myocarde de l'adulte en réponse à l'établissement d'une insuffisance cardiaque (Yin et al. 2014). D'autre part une augmentation de l'expression de myh7 est notamment démontrée dans la DMD (Murphy et al. 2016) et dans la cardiomyopathie arythmogène qui se caractérisent toutes deux par une perte de cardiomyocyte et l'installation d'une fibrose du myocarde (Gerçek 2017).
- BMP4 : la protéine morphogénétique osseuse 4 (BMP4) est un répresseur important de la différenciation myogénique. Il interfère avec le processus de régénération musculaire dans la DMD (Shi 2011).

L'analyse de marqueurs moléculaires liés à l'installation de la fibrose cardiaque dans la DMD confirme une augmentation significative de l'expression génique de CTGF et collagène I (Col1a1) chez les animaux mdx par rapport aux animaux sains (respectivement p<0,001 et p<0,05, test t non-apparié, figure 3A). Le traitement de deux mois par BIO101 à la dose de 50 mg/kg*jour prévient (p=0,0506, test t non-apparié) l'expression génique de CTGF chez les animaux mdx traités par BIO101 par rapport aux animaux mdx contrôles, ayant reçu le véhicule (Figure 3B). Le traitement d'animaux mdx par BIO103 à la même dose tend à prévenir l'expression génique de Col1a1 par rapport aux animaux mdx contrôles (figure 3B).

L'expression génique myh7, est significativement augmentée (p<0.05, test t non-apparié) chez les animaux mdx par rapport aux animaux sauvages C57 (Figure 3C). Le traitement des animaux mdx par BIO101 et BIO103 à la dose de 50 mg/kg*jour réduit de manière significative l'expression de myh7 (p<0,01, test t non-apparié) par rapport aux animaux mdx ayant reçu le véhicule (Figure 3D). Par ailleurs, BIO101 réduit l'expression génique de BMP4 (p=0,0529, test t non-apparié) par rapport aux animaux mdx contrôles, ayant reçu le véhicule (Figure 3D).

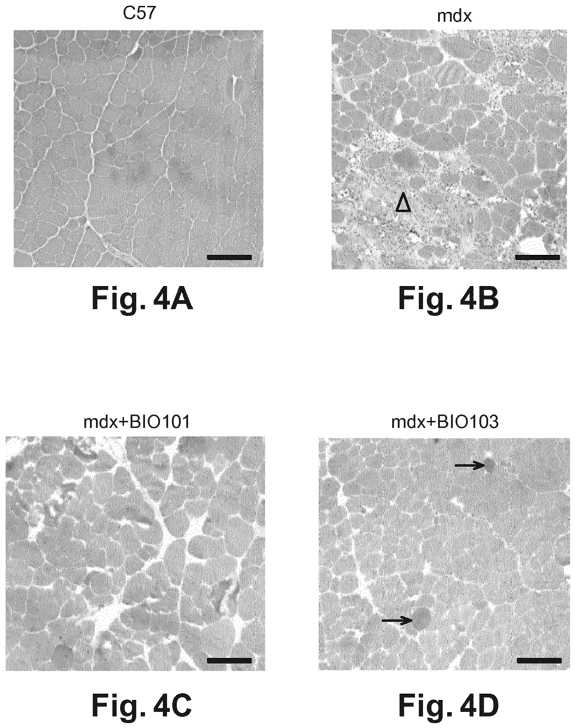

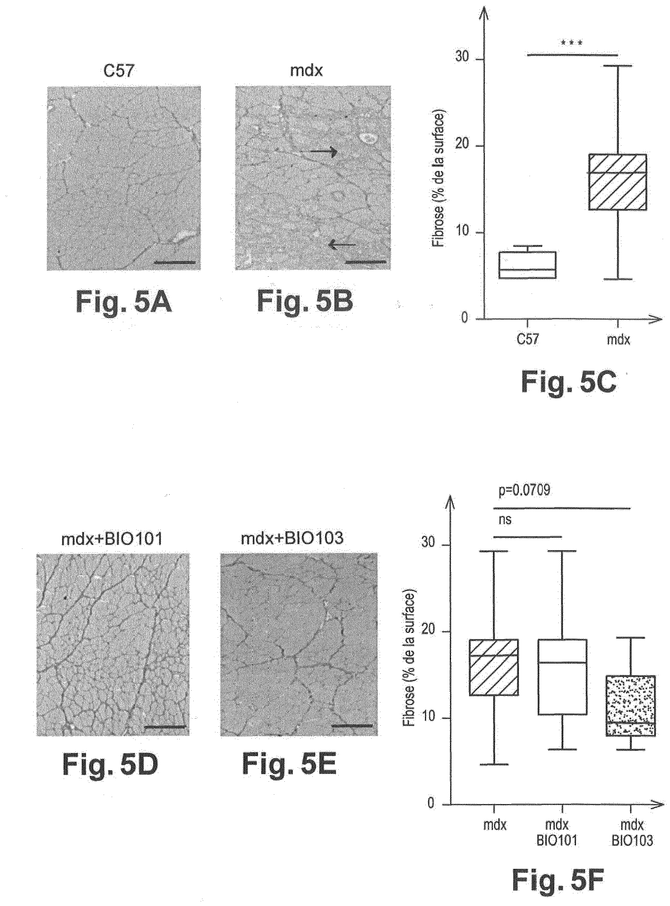

### Fibrose du muscle squelettique (étude histologique : figures 4 et 5)

Une évaluation de la fibrose musculaire par étude histologique est réalisée sur le muscle tibial antérieur (abrévié TA). Des coupes histologiques (7µm) sont réalisées et colorées soit à l'hématoxyline éosine (HE) soit au rouge sirius (RS).

Une étude anatomopathologique des coupes de muscle tibial antérieur colorées par HE permet d'évaluer le niveau d'affection des fibres musculaires. Des images représentatives des coupes de muscle tibial antérieur colorées au HE provenant des animaux C57 (Figure 4A), mdx (Figure 4B), mdx traités par BIO101 (Figure 4C) et mdx traités par BIO103 (Figure 4D) sont présentées. Les barres d'échelles correspondent à 200µm. Les muscles TA de souris C57 ne présentent aucune lésion musculaire (Figure 4A). Les TA des souris mdx non traités présentent une grande diversité de profils lésionnels : une anisocytose modérée avec de nombreuses fibres nécrotiques. Certains muscles présentent quant à eux, une anisocytose marquée à sévère, multifocale, avec une atrophie d'une grande partie des myocytes, ainsi que de volumineux foyers inflammatoires chroniques associant de la fibrose (Figure 4B) et des infiltrats de cellules mononucléées (macrophages principalement). Le signe Δ indique les foyers inflammatoires auxquels est associé de la fibrose. Enfin, certains muscles présentent de larges plages de nécrose aigue. Les muscles des souris traitées par BIO101 présentent exclusivement deux types de profil lésionnels : un profil lésionnel léger avec peu d'anisocytose, de nécrose ou d'inflammation (37,5%, 3 sur 8 des TA) et un profil lésionnel marqué avec une anisocytose, des fibres nécrotiques dispersées et une inflammation variable (62,5%, 5 sur 8 des TA) (Figure 4C). De manière intéressante, les muscles des souris traitées par BIO103 présentent moins de lésions musculaires avec un profil lésionnel léger. En effet, 67% (6 sur 9 des TA) des muscles traités par BIO103 peuvent être classifiés dans cette catégorie. D'un point de vue histologique, la grande majorité des myocytes présente des noyaux centraux (témoins de phénomènes de nécrose-régénération), une variation de taille (anisocytose) minime à légère, de rares myocytes nécrotiques et hyper-contractés (phase initiale de nécrose ; flèches noires, Figure 4D), et de rares cellules inflammatoires dans l'endomysium. Les autres muscles traités avec BIO103 présentent soit un profil lésionnel marqué de tendance plutôt nécrotique (11% des TA, 1 sur 9 des TA) avec une anisocytose, des fibres nécrotiques dispersées, et une nécrose aigüe soit un profil lésionnel marqué tendant vers l'atrophie avec une forte anisocytose, sans fibre nécrotique (11%, 1 sur 9 des TA). Enfin, un des TA présente un profil lésionnel sévère avec de grandes plages de nécrose aiguë sans régénération (11%, 1 sur 9 des TA).

Une analyse anatomopathologique des coupes de muscle tibial antérieur colorées par RS permet de quantifier la surface de fibrose. La coloration RS permet de visualiser les zones de fibrose sur les coupes histologiques (Rittié 2017). Des images représentatives des coupes de muscle tibial antérieur colorées au RS provenant des animaux C57 (Figure 5A), mdx (Figure 5B), mdx traités par BIO101 (Figure 5D) et C57 mdx traités par BIO103 (Figure 5E) sont présentées. La barre d'échelle représente 200µm. Le pourcentage de surface correspondant à la fibrose est significativement augmenté (p<0,001, test de Mann Whitney) dans le groupe mdx par rapport au groupe C57 (Figure 5C). Les flèches noires indiquent les zones présentant une forte fibrose musculaire. Le traitement des animaux mdx par BIO103 tend nettement à prévenir (p=0,0709, test t non apparié) l'apparition de fibrose par rapport au groupe mdx contrôle, ayant reçu le véhicule (Figure 5F).

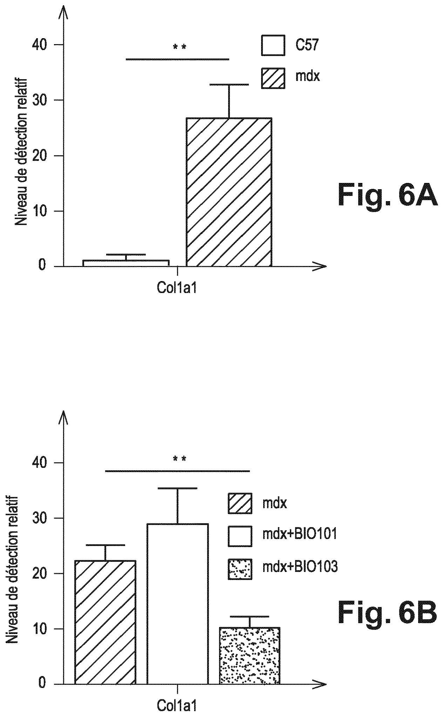

### Fibrose du muscle squelettique (étude biochimique : figure 6)

De manière complémentaire aux observations faites sur le muscle tibial antérieur, une évaluation d'un marqueur de fibrose musculaire est réalisée sur le muscle gastrocnémien. Le muscle est dissocié sous agitation modérée pendant 16h à 4°C dans du tampon de lyse RIPA. Les protéines sont dosées, résolues en gel SDS PAGE avant d'être transférées sur membrane PVDF. Le collagène I est détecté par western blot, révélé par chimioluminescence puis quantifié par densitométrie après normalisation par rapport à la détection de la GAPDH. De manière attendue, l'expression protéique du collagène I est significativement augmentée (p<0,01 ; test t non apparié) dans les muscles gastrocnémiens des souris mdx par rapport aux muscles des souris saines (C57). L'expression protéique du collagène I est augmentée de 27 fois (Figure 6A). Le traitement par BIO103 des souris mdx permet d'observer une diminution significative (p<0,01 ; test t non apparié) de l'expression protéique du collagène I par rapport aux souris mdx non-traitées (véhicule) (Figure 6B).

D'autres résultats expérimentaux concernant l'activité biologique de BIO101 et BIO103 sont présentés au point 5 de la présente description, ci-après.

### 4. Activité biologique in vitro de BIO101

### Différenciation des myoblastes en myotubes

Sterrenburg et ses collaborateurs ont montré qu'une régénération musculaire inefficace dans les cellules DMD est causée par une différenciation des myoblastes altérée et un maintien déficient des myotubes (Turk et al., 2006). Une combinaison de prolifération réduite, de fusion altérée et de maintenance déficiente des myotubes DMD conduit à une régénération musculaire inefficace et peut contribuer au phénotype sévère des patients DMD.

Divers paramètres ont été mesurés pour apprécier la différenciation des myotubes. Les cellules humaines du muscle squelettique DMD (KM571 DMD10FL Cl1) ont été mises en différenciation pendant 3 jours puis incubées 3 jours avec ou sans BIO101 à 10 µM. L'indice de fusion et le nombre de noyaux par myotube ont été validés en tant qu'indicateurs pertinents et sensibles de la différenciation du myotube.

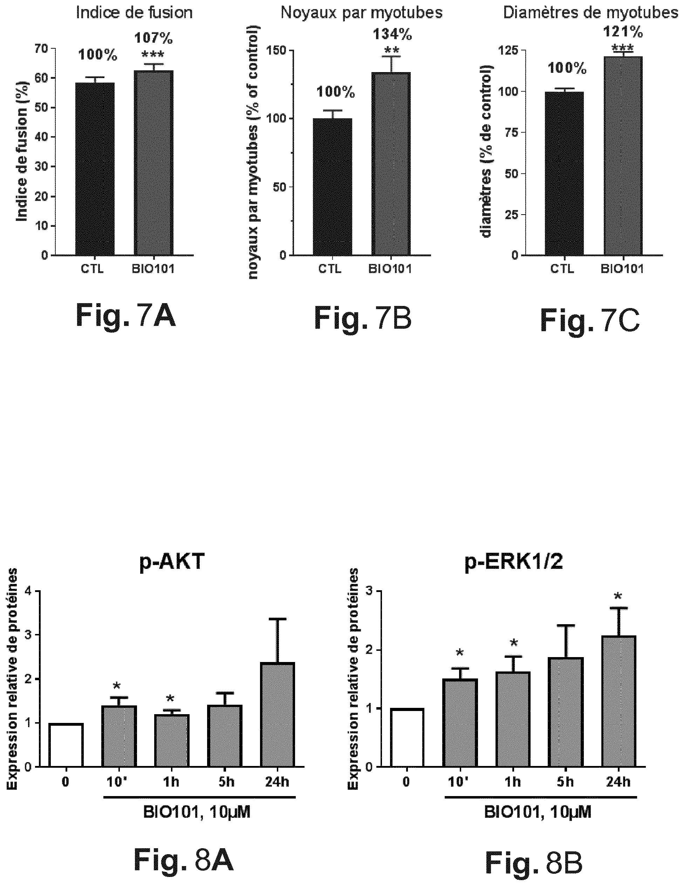

L'indice de fusion illustré en figure 7A représente le pourcentage de noyaux de cellules différenciées par rapport au total des cellules.

Le nombre de noyaux par myotubes est illustré en figure 7B, 300 myotubes ont été comptés.

Le diamètre des myotubes est illustrée en figure 7C, 150 myotubes ont été mesurés par condition.

On observe en présence de BIO101 une différenciation améliorée des myoblastes DMD humains en myotubes (figures 7A, 7B et 7C). Une augmentation significative de +7% (p < 0.001) de l'indice de fusion est observée.

Celle-ci s'accompagne d'un accroissement de 34% (p < 0.01) du nombre de noyaux par myotube, et d'un accroissement de 21% (p < 0.001) du diamètre des myotubes.

Les résultats en figures 7A, 7B et 7C présentent la moyenne des données mesurées lors de trois expériences indépendantes, plus ou moins l'erreur type (SEM). L'analyse statistique par un test de Mann-Whitney montre une différence significative entre les cellules traitées avec BIO101 à une concentration de 10 µM (micromolaires) et les cellules contrôle non traitées (annoté « CTL » en figure 7), p<0,01 (**); p<0.001 (***).

### Voies de signalisation

La voie de signalisation PI3K/AKT/mTOR joue un rôle essentiel dans la croissance cellulaire, la prolifération, la motilité, la survie, l'apoptose, l'autophagie et l'angiogenèse (Hay et al., 2004).

Les MAP kinases (abrévié de l'anglais mitogen-activated protein kinases), y compris ERK1 et ERK2, participent à une variété de fonctions telles que la régénération musculaire, le remodelage et les contractions. Chez les souris mdx, il a été démontré (Roffe et al., 2010) que l'activation des voies PI3K/Akt et MAPK dans les cellules musculaires était notamment associée à des effets bénéfiques (Turgeman et al., 2008 ; Huebner et al., 2008).

Les cellules humaines du muscle squelettique DMD (KM571 DMD10FL Cl1) ont été mises en différenciation pendant 5 jours puis incubées ou non avec BIO101 à 10 µM pendant des périodes comprises entre 10 minutes et 24 heures.

Une analyse densitométrique de résultats d'au moins 6 expériences indépendantes de Western blot décrivant les effets de BIO101 sur la phosphorylation d'AKT (Figure 8A) et ERK1/2 (Figure 8B) est présentée, *p <0,05 versus le contrôle non traité.

On constate que BIO101 induit une augmentation significative de p-AKT et de p-ERK1/2.

A la lecture des résultats présentés on constate que BIO101 induit une activation importante et précoce des voies de signalisation AKT et MAPK dans les myocytes DMD humains.

### Respiration cellulaire

Il est connu qu'il existe une diminution de la respiration basale mitochondriale dans les fibres musculaires DMD (Schuh et al. 2015).

De manière très intéressante, sur des myotubes de patients DMD, les propriétés bénéfiques de BIO101 au niveau de la différenciation des myoblastes d'un patient DMD (Figure 7) s'accompagnent d'effets positifs sur le métabolisme énergétique.

Les cellules musculaires squelettiques humaines DMD (KM571 DMD10FL Cl1) ont été différenciées pendant 4 jours puis incubées en présence ou en l'absence de BIO101 (1 ou 5 µM) pendant 2 jours. La vitesse de consommation d'oxygène aussi appelée OCR (abrévié de l'anglais Oxygen Consumption Rate), qui reflète la respiration mitochondriale des cellules a été mesurée grâce au Seahorse XF Analyzer (marque déposée, Agilent).

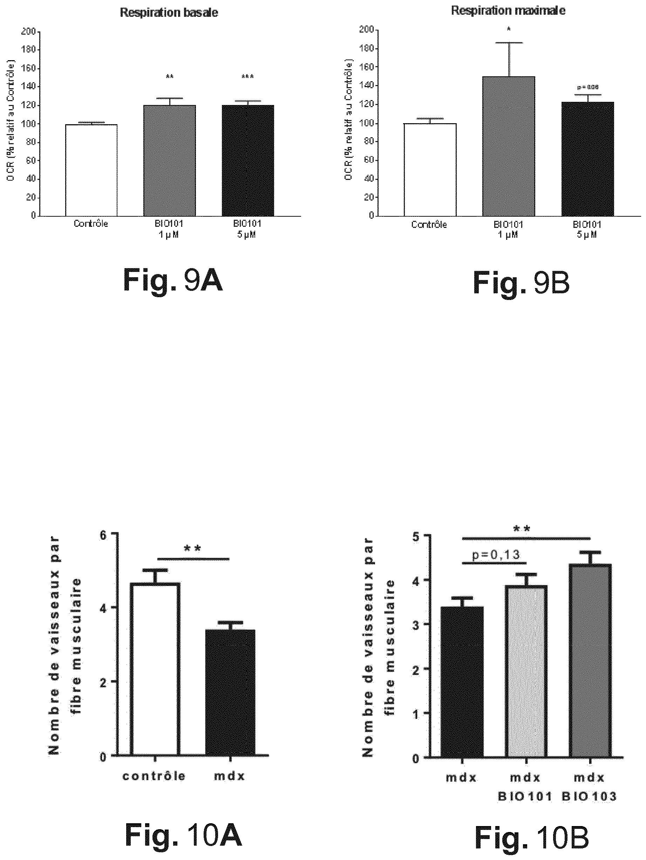

La respiration basale est mesurée directement alors que la respiration maximale est mesurée après l'addition d'oligomycine suivie de FCCP (figure 9A et figure 9B). Les résultats sont rapportés à l'OCR des cellules non traitées et présentés sous forme de moyennes plus ou moins l'erreur type (SEM).

Après deux jours de traitement par BIO101, il apparait que les cellules provenant d'un patient DMD ont des respirations mitochondriales basale et maximale significativement augmentées par rapport à celles des cellules non traitées.

Plus précisément on constate une respiration basale augmentée de 20% (p < 0.01) sur les cellules traitées par BIO101 à une dose de 1 µM. On constate une respiration basale augmentée de 21% (p < 0.001) sur les cellules traitées par BIO101 à une dose de 5 µM. On constate une respiration maximale augmentée de 51 % (p<0.05) sur les cellules traitées par BIO101 à une dose de 1 µM. On constate une respiration maximale augmentée de 22 % (p = 0.13) sur les cellules traitées par BIO101 à une dose de 5 µM.

Les résultats présentés sont issus d'au moins quatre expérimentations indépendantes.

Le test statistique Mann et Whitney a permis de montrer une différence significative entre les cellules traitées avec BIO101 à 1 et 5 µM et les cellules non traitées, p<0.05 (*), <0.01 (**), 0.001 (***).

BIO101 contribue à l'amélioration du métabolisme énergétique des cellules musculaires de patient atteint de DMD.

### 5. Activité biologique in vivo de BIO101 et BIO103

Le muscle strié squelettique est l'un des tissus les plus vascularisés de l'organisme et les cellules endothéliales sont essentielles aux processus de régénération musculaire. La dystrophine est présente au niveau des cellules musculaires lisses des vaisseaux et son absence peut causer des troubles de la vascularisation. En effet, il a été montré que dans un muscle DMD les fibres nécrotiques sont très souvent groupées et que ce phénomène était dû à une réduction du débit sanguin des capillaires communs à ce groupe de fibres (Rando, 2001) provoquant une nécrose ischémique des fibres.

Outre le débit sanguin, des études plus récentes ont démontré une réduction de la densité vasculaire chez des souris mdx (Loufrani et al., 2004 ; Matsakas et al., 2013). Une autre étude a également pu mettre en évidence que l'angiogenèse était altérée dans le modèle de souris mdx (Palladino et al., 2013). Ces études montrent qu'il existe un rationnel pour penser qu'il existe un défaut de vascularisation dans la DMD que ce soit en termes de sa qualité, de sa quantité ainsi qu'au niveau de l'angiogenèse, et que le tissu musculaire se trouve dans des conditions ischémiques.

### Vascularisation du muscle squelettique

Une étude quantitative de la vascularisation a été réalisée sur des coupes de muscle tibial antérieur (abrévié TA) de souris saines C57 ainsi que sur des souris mdx traitées avec le véhicule et des souris mdx des souris mdx traitées BIO101 ou BIO103.

Un double marquage par immunofluorescence est réalisé, un marquage anti-laminine ayant pour fonction d'identifier la membrane basale des fibres musculaires et un marquage anti-CD31 ayant pour fonction d'identifier les vaisseaux. Le nombre de vaisseaux par fibre musculaire est quantifié.

De manière attendue, il est constaté que les animaux qui présentent une mutation sur le gène de la dystrophine (mdx) ont un nombre de vaisseaux par fibre musculaire réduit. La réduction constatée est de 27,2% (p<0.01) par rapport aux animaux sains (C57) (Figure 10A).

Les animaux mdx ont reçu quotidiennement, par voie orale, soit le véhicule soit BIO101 (50mg/kg*jour) soit BIO103 (50mg/kg*jour). Les résultats de la quantification du nombre de fibres musculaire après deux mois d'exposition quotidienne sont présentés en Figure 10B.

On constate que les animaux mdx qui ont reçu BIO101 ou BIO103 présentent une vascularisation plus importante que les animaux mdx qui ont reçu le véhicule. On constate que BIO101 tend à améliorer le nombre vaisseaux par fibre musculaire par une augmentation de 14,1% (p=0,13, test t non apparié). De manière plus marquée, BIO103 augmente significativement, de 28,2% (p=0,006, test t non apparié) la vascularisation musculaire par rapport aux animaux mdx non traités (véhicule).

Les traitements par BIO101 et surtout par BIO103 augmentent le nombre de vaisseaux par fibre musculaire.

Ces résultats démontrent une efficacité de BIO101 et BIO103 sur différents paramètres musculaires *in vitro* et *in vivo.* En effet, le traitement de cellules musculaires de patients atteints de DMD par BIO101 ou BIO103 montrent qu'ils améliorent la différenciation, et le métabolisme énergétique (respiration mitochondriale) des cellules et qu'ils sont capables d'augmenter la vascularisation musculaire *in vivo,* importante pour un bon fonctionnement musculaire.

### Conclusion

Compte tenu des propriétés de BIO101 et de BIO103 sur la fonction musculaire et sur l'établissement de la fibrose du myocarde et du muscle squelettique de mammifères atteints par une myopathie, l'utilisation de BIO101 et de BIO103 peut donc être proposée, seule ou en complément d'un traitement visant à corriger les effets d'une altération génique, pour préserver la fonction musculaire, notamment la force musculaire et la tolérance à l'effort et ainsi ralentir l'évolution de myopathies qui ont pour conséquence la détérioration de ladite fonction musculaire, et plus particulièrement l'installation d'une fibrose. Les myopathies concernées par la présente invention incluent, les myopathies génétiques, notamment la dystrophie musculaire de Duchenne.

### Bibliographie

Barnabei M.S., Martindale J.M., Townsend D., Metzger J.M. (2011). Exercise and muscular dystrophy: implications and analysis of effects on musculoskeletal and cardiovascular systems. Compr Physiol. 2011 Jul;1(3):1353-63
Brigstock DR 2010. Connective tissue growth factor (CCN2, CTGF) and organ fibrosis: lessons from transgenic animais. J Cell Commun Signal. 4 (1): 1-4**;**
Bulfield G., Siller W. G., Wight P. A., Moore K. J. (1984). X chromosome-linked muscular dystrophy (mdx) in the mouse. Proc. Natl. Acad. Sci. USA 81, 1189-1192.
Ferry A, Amiridis I, Rieu M. 1992. Glycogen depletion and resynthesis in the rat after downhill running. Eur J Appl Physiol Occup Physiol 64(1): 32-35.
Ferry A, Rieu P, Le Page C, Elhabazi A, Laziri F, Rieu M. 1993. Effect of physical exhaustion and glucocorticoids (dexamethasone) on T-cells of trained rats. Eur J Appl Physiol Occup Physiol 66(5): 455-460.
Gorelick-Feldman J, MacLean D, Ilic N, Poulev A, Lila MA, Cheng D, Raskin I. 2008. Phytoecdysteroids increase protein synthesis in skeletal muscle cells. J Agric Food Chem56: 3532-3537.
Hadj-Said W, Bangratz M, Vignaud A, Chatonnet A, Butler-Browne G, Nicole S, Agbulut O, Ferry A. 2012. Effect of locomotor training on muscle performance in the context of nerve-muscle communication dysfunction. Muscle Nerve 45(4): 567-577.
Hay N and Sonenberg N. (2004). Upstream and downstream of mTOR. Genes Development 18: 1926-1945.
Huebner KD, Jassal DS, Halevy O, Pines M and Anderson JE. (2008). Functional resolution of fibrosis in mdx mouse dystrophic heart and skeletal muscle by halofuginone. Am J Physiol Heart Circ Physiol. 294(4): H1550-61.
Lafont R, Raynal S, Dioh W, Veillet S, Lepifre F, Durand JD. 2014. Produits derives de la 20-hydroxyecdysone et leur utilisation dans la préparation de médicaments. Demande FR3021318 (déposé le 20/05/2014).
Lawrence MM. 2012. Ajuga turkestanica as a countermeasure against sarcopenia and dynapenia. Ms thesis, Appalachian State University.
Loufrani L, Dubroca C, You D, Li Z, Levy B, Paulin D et al. (2004). Absence of dystrophin in mice reduces NO-dependent vascular function and vascular density: total recovery after a treatment with the amino-glycoside gentamicin. Arterioscler. Thromb. Vasc. Biol. 24: 671-676.
Matsakas A, Yadav V, Lorca S, Narkar V. (2013). Muscle ERR gamma mitigates Duchenne muscular dystrophy via metabolic and angiogenic reprogramming. FASEB J. 27: 4004-4016.
Murphy S., Dowling P., Zweyer M., Mundegar R., Henry M., Meleady P., Swandulla D., Ohlendieck K. (2016). Proteomic analysis of dystrophin deficiency and associated changes in the aged mdx-4cv heart model of dystrophinopathy-related cardiomyopathy. J. Proteomics, 145, 24-36
Palladino M, Gatto I, Neri V, Straino S, Smith RC, Silver M et al. (2013). Angiogenic impairment of the vascular endothelium: a novel mechanism and potential therapeutic target in muscular dystrophy. Arterioscler. Thromb. Vasc. Biol. 33: 2867-2876.Rando TA. (2001). Role of nitric oxide in the pathogenesis of muscular dystrophies: a "two hit" hypothesis of the cause of muscle necrosis. Microsc. Res. Tech. 55: 223-235.
Rittié L. (2017). Method for Picrosirius Red-Polarization Détection of Collagen Fibers in Tissue Sections. Methods Mol Biol. 1627: 395-407.
Roffe S, Hagai Y, Pines M, Halevy O. (2010). Halofuginone inhibits Smad3 phosphorylation via the PI3K/Akt and MAPK/ERK pathways in muscle cells: effect on myotube fusion. Exper Cell Res. 316(6): 1061-1069.
Schuh RA, Jackson KC, Khairallah RJ, Ward CW, Spangenburg EE. (2012). Measuring mitochondrial respiration in intact single muscle fibers. Am J Physiol Regul Integr Comp Physiol. 302(6): R712-R719.
Shi S., Hoogaars W.M., de Gorter D.J., van Heiningen S.H., Lin H.Y., Hong C.C., Kemaladewi D.U., Aartsma-Rus A., ten Dijke P., 't Hoen P.A. (2011). BMP antagonists enhance myogenic differentiation and ameliorate the dystrophic phenotype in a DMD mouse model. Neurobiol Diseases 41(2), 353-360
Sicinski P, Geng Y, Ryder-Cook AS, Barnard EA, Darlison MG, Barnard PJ.1989. The molecular basis of muscular dystrophy in the mdx mouse: a point mutation. Science 244(4912), 1578-1580.
Simakin SYu, Panyushkin VV, Portugalov SN, Kostina LV, Martisorov EG. 1988. Combined application of préparation Ecdysten. Science Bulletin N °2, 29-31.
Song Y, Yao S, Liu Y, Long L, Yang H, Li Q, Liang J, Li X, Lu Y, Zhu H, Zhang N. 2017. Expression levels of TGF-β1 and CTGF are associated with the severity of Duchenne muscular dystrophy. Exp Ther Med 13(4): 1209-1214.
Syrov VN. 2000. Comparative experimental investigations of the anabolic activity of ecdysteroids and steranabols. Pharm Chem Journal 34(4):193-197.
Tóth N, Szabó A, Kacsala P, Héger J, Zádor E. 2008. 20-Hydroxyecdysone increases fiber size in a muscle-specific fashion in rat. Phytomedicine 15: 691-698.
Turgeman T, Hagai Y, Huebner K, Jassal DS, Anderson JE, Genin O, Nagler A, Halevy O, Pines M. (2008). Prévention of muscle fibrosis and improvement in muscle performance in the mdx mouse by halofuginone. Neuromuscul Disord. 18(11): 857-868
Turk R, Sterrenburg E, van der Wees CG, de Meijer EJ, de Menezes RX, Groh S, Campbell KP, Noguchi S, van Ommen GJ, den Dunnen JT, 't Hoen PA. (2006). Common pathological mechanisms in mouse models for muscular dystrophies. FASEB J. 20(1): 127-129.Wang X, McLennan SV, Allen TJ, Twigg SM. 2010. Régulation of pro-inflammatory and pro-fibrotic factors by CCN2/CTGF in H9c2 cardiomyocytes. J Cell Comm. Signal 4: 15-23
Yin Z., Ren J., Guo W. (2014), Sarcomeric protein isoform transitions in cardiac muscle: A journey to heart failure. Biochim Biophys Acta. 1852(1):47-52

## Revendications

1. 20-hydroxyecdysone et ses dérivés, pour utilisation dans le traitement d'une myopathie résultant d'une altération génétique incluant une mutation du gène de la dystrophine.

2. 20-hydroxyecdysone et ses dérivés, pour utilisation selon la revendication précédente, pour le traitement d'affections résultant d'une altération de la fonction musculaire provoquée par une myopathie génétique.

3. 20-hydroxyecdysone et ses dérivés pour utilisation selon la revendication précédente, **caractérisée en ce que** la fonction musculaire est celle du muscle strié squelettique ou du myocarde.

4. 20-hydroxyecdysone et ses dérivés pour utilisation selon la revendication précédente, dans laquelle l'altération de la fonction musculaire est une hypertrophie du myocarde.

5. 20-hydroxyecdysone et ses dérivés pour utilisation selon l'une quelconque des revendications précédentes, **caractérisé en ce que** la fonction musculaire est au moins en partie altérée par l'installation progressive d'une fibrose.

6. 20-hydroxyecdysone et ses dérivés, pour utilisation selon l'une quelconque des revendications précédentes, **caractérisée en ce que** la myopathie résultant d'une altération génétique est une dystrophie musculaire de Duchenne (DMD) et/ou une dystrophie musculaire de Becker (BMD).

7. 20-hydroxyecdysone et ses dérivés destinés à être utilisés selon l'une quelconque des revendications précédentes, **caractérisé en ce que** la respiration mitochondriale des cellules musculaires est augmentée.

8. 20-hydroxyecdysone et ses dérivés pour utilisation selon l'une quelconque des revendications précédentes, **caractérisé en ce que** le nombre de vaisseaux par fibre musculaire est augmenté.

9. 20-hydroxyecdysone et ses dérivés pour utilisation selon l'une quelconque des revendications précédentes, **caractérisé en ce que** la différenciation des myoblastes en myotubes est augmentée.

10. 20-hydroxyecdysone et ses dérivés pour utilisation selon l'une quelconque des revendications précédentes, **caractérisés en ce que** la 20-hydroxyecdysone est un composé de formule (I):

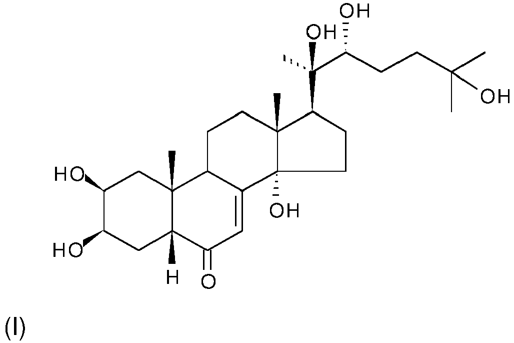

11. 20-hydroxyecdysone et ses dérivés pour utilisation selon l'une des revendication précédentes, **caractérisé en ce que** la 20-hydroxyecdysone est un extrait de plante ou d'une partie de plante, ladite plante étant choisie parmi les végétaux contenant au moins 0,5% de 20-hydroxyecdysone de formule (I), en poids sec dudit végétal, ledit extrait comportant au moins 95%, et de préférence au moins 97%, de 20-hydroxyecdysone de formule (I).

12. 20-hydroxyecdysone et ses dérivés pour utilisation selon la revendication précédente, **caractérisé en ce que** l'extrait comporte entre 0 et 0,05 % en poids sec de l'extrait d'impuretés susceptibles d'affecter l'innocuité, la disponibilité ou l'efficacité d'une application pharmaceutique dudit extrait.

13. 20-hydroxyecdysone et ses dérivés pour utilisation selon l'une quelconque des revendications précédentes **caractérisé en ce que** la plante est choisie parmi *Stemmacantha carthamoides, Cyanotis arachnoidea* et *Cyanotis vaga.*

14. 20-hydroxyecdysone et ses dérivés pour utilisation selon l'une quelconque des revendications précédentes, **caractérisé en ce que** la 20-hydroxyecdysone est un extrait de racines de *Stemmacantha carthamoides* comportant au moins 95%, et de préférence au moins 97%, de 20-hydroxyecdysone de formule (I).

15. 20-hydroxyecdysone et ses dérivés pour utilisation selon l'une quelconque des revendications précédentes, **caractérisé en ce qu'**un dérivé est un composé de formule générale (II) : 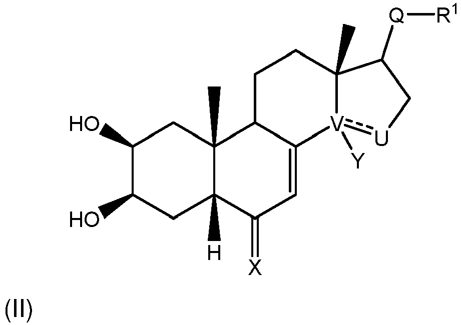 dans laquelle :
**V-U** est une liaison simple carbone-carbone et **Y** est un groupement hydroxyle ou un hydrogène, ou **V-U** est une liaison éthylénique C=C ;
**X** est un oxygène,
**Q** est un groupement carbonyle ;
**R¹** est choisi parmi : un groupement (C₁-C₆)**W**(C₁-C₆) ; un groupement (C₁-C₆)**W**(C₁-C₆)**W**(C₁-C₆) ; un groupement (C₁-C₆)**W**(C₁-C₆)CO₂(C₁-C₆); un groupement (C₁-C₆)**A**, **A** représentant un hétérocycle éventuellement substitué par un groupement de type OH, OMe, (C₁-C₆), N(C₁-C₆), CO₂(C₁-C₆); un groupement CH₂Br;
**W** étant un hétéroatome choisi parmi N, O et S.

16. 20-hydroxyecdysone et ses dérivés pour utilisation selon la revendication précédente, **caractérisé en ce que** dans la formule (II) :
**Y** est un groupement hydroxyle ;
**R¹** est choisi parmi : un groupement (C₁-C₆)**W**(C₁-C₆) ; un groupement (C₁-C₆)**W**(C₁-C₆)**W**(C₁-C₆); un groupement (C₁-C₆)**W**(C₁-C₆)CO₂(C₁-C₆); un groupement (C₁-C₆)**A**, **A** représentant un hétérocycle éventuellement substitué par un groupement de type OH, OMe, (C₁-C₆), N(C₁-C₆), CO₂(C₁-C₆).
**W** étant un hétéroatome choisi parmi N, O et S.

17. 20-hydroxyecdysone et ses dérivés pour utilisation selon l'une quelconque des revendications précédentes, **caractérisé en ce qu'**ils sont choisis parmi :
- **n°1 :** (2S,3R,5R,10R,13R,14S,17S)-2,3,14-trihydroxy-10,13-diméthyl-17-(2-morpholinoacétyl)-2,3,4,5,9,11,12,15,16,17-décahydro-1H-cyclopenta[a]phénanthrèn-6-one;
- **n°2 :** (2S,3R,5R,10R,13R,14S,17S)-2,3,14-trihydroxy-17-[2-(3-hydroxypyrrolidin-1-yl)acétyl]-10,13-diméthyl-2,3,4,5,9,11,12,15,16,17-décahydro-1H-cyclopenta[a]phénanthrèn-6-one;
- **n°3 :** (2S,3R,5R,10R,13R,14S,17S)-2,3,14-trihydroxy-17-[2-(4-hydroxy-1-pipéridyl)acétyl]-10,13-diméthyl-2,3,4,5,9,11,12,15,16,17-décahydro-1H-cyclopenta[a]phénanthrèn-6-one;
- **n°4 :** (2S,3R,5R,10R,13R,14S,17S)-2,3,14-trihydroxy-17-[2-[4-(2-hydroxyéthyl)-1-pipéridyl]acétyl]-10,13-diméthyl-2,3,4,5,9,11,12,15,16,17-décahydro-1H-cyclopenta[a]phénanthrèn-6-one;
- **n°5 :** (2S,3R,5R,10R,13R,14S,17S)-17-[2-(3-diméthylaminopropyl(méthyl)amino)acétyl]-2,3,14-trihydroxy-10,13-diméthyl-2,3,4,5,9,11,12,15,16,17-décahydro-1H-cyclopenta[a]phénanthrèn-6-one;
- **n°6 :** 2-[2-oxo-2-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]sulfanylacétate d'éthyle;
- **n°7 :** (2S,3R,5R,10R,13R,14S,17S)-17-(2-éthylsulfanylacétyl)-2,3,14-trihydroxy-10,13-diméthyl-2,3,4,5,9,11,12,15,16,17-décahydro-1H-cyclopenta[a]phénanthrèn-6-one;
- **n°8** : (2S,3R,5R,10R,13R,14S,17S)-2,3,14-trihydroxy-17-[2-(2-hydroxyéthylsulfanyl)acétyl]-10,13-diméthyl-2,3,4,5,9,11,12,15,16,17-décahydro-1H-cyclopenta[a]phénanthrèn-6-one.

18. 20-hydroxyecdysone et ses dérivés pour utilisation selon l'une quelconque des revendications précédentes, **caractérisé en ce que** le dérivé est de formule (III) :

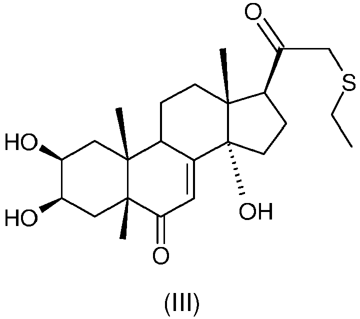

## Patentansprüche

1. 20-Hydroxyecdyson und dessen Derivate zur Verwendung bei der Behandlung einer Myopathie, die aus einer genetischen Veränderung resultiert, die eine Mutation des Dystrophin-Gens einschließt.

2. 20-Hydroxyecdyson und dessen Derivate zur Verwendung nach vorangehendem Anspruch zur Behandlung von Erkrankungen die aus einer Veränderung der Muskelfunktion resultiert, die von einer genetischen Myopathie hervorgerufen wird.

3. 20-Hydroxyecdyson und dessen Derivate zur Verwendung nach vorangehendem Anspruch, **dadurch gekennzeichnet, dass** die Muskelfunktion die der gestreiften Skelettmuskulatur oder des Herzmuskels ist.

4. 20-Hydroxyecdyson und dessen Derivate zur Verwendung nach vorangehendem Anspruch, wobei die Veränderung der Muskelfunktion eine Hypertrophie des Herzmuskels ist.

5. 20-Hydroxyecdyson und dessen Derivate zur Verwendung nach einem der vorangehenden Ansprüche, **dadurch gekennzeichnet, dass** die Muskelfunktion mindestens teileweise durch die fortschreitende Ausbildung einer Fibrose verändert ist.

6. 20-Hydroxyecdyson und dessen Derivate zur Verwendung nach einem der vorangehenden Ansprüche, **dadurch gekennzeichnet, dass** die Myopathie, die aus einer genetischen Veränderung resultiert, eine Duchenne-Muskeldystrophie (DMD) und/oder eine Becker-Muskeldystrophie (BMD) ist.

7. 20-Hydroxyecdyson und dessen Derivate, die bestimmt sind, nach einem der vorangehenden Ansprüche verwendet zu werden, **dadurch gekennzeichnet**, die Mitrochondrienatmung der Muskelzellen erhöht wird.

8. 20-Hydroxyecdyson und dessen Derivate zur Verwendung nach einem der vorangehenden Ansprüche, **dadurch gekennzeichnet, dass** die Anzahl der Gefäße je Muskelfaser erhöht wird.

9. 20-Hydroxyecdyson und dessen Derivate zur Verwendung nach einem der vorangehenden Ansprüche, **dadurch gekennzeichnet, dass** die Differenzierung der Myoblasten in den Myotuben erhöht wird.

10. 20-Hydroxyecdyson und dessen Derivate zur Verwendung nach einem der vorangehenden Ansprüche, **dadurch gekennzeichnet, dass** das 20-Hydroxyecdyson eine Verbindung der Formel (I) ist:

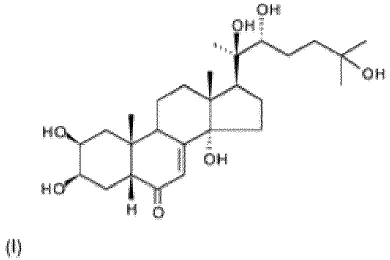

11. 20-Hydroxyecdyson und dessen Derivate zur Verwendung nach einem der vorangehenden Ansprüche, **dadurch gekennzeichnet, dass** das 20-Hydroxyecdyson ein Extrakt aus einer Pflanze oder aus einem Pflanzenteil ist, wobei die Pflanze aus den Pflanzen ausgewählt ist, die mindestens 0,5 % Trockengewicht der Pflanze 20-Hydroxyecdyson der Formel (I) enthalten, wobei der Extrakt mindestens 95 %, vorzugsweise mindestens 97 % 20-Hydroxyecdyson der Formel (I) enthält.

12. 20-Hydroxyecdyson und dessen Derivate zur Verwendung nach vorangehendem Anspruch, **dadurch gekennzeichnet, dass** der Extrakt zwischen 0 und 0,05 % Trockengewicht des Extrakts Unreinheiten aufweist, die imstande sind, die Unbedenklichkeit, die Verfügbarkeit oder die Wirksamkeit einer pharmazeutischen Anwendung des Extrakts zu beeinträchtigen.

13. 20-Hydroxyecdyson und dessen Derivate zur Verwendung nach einem der vorangehenden Ansprüche **dadurch gekennzeichnet, dass** die Pflanze aus *Stemmacantha carthamoides, Cyanotis arachnoidea* und *Cyanotis vaga* ausgewählt ist.

14. 20-Hydroxyecdyson und dessen Derivate zur Verwendung nach einem der vorangehenden Ansprüche, **dadurch gekennzeichnet, dass** das 20-Hydroxyecdyson ein Extrakt aus den Wurzeln von *Stemmacantha carthamoides* ist, der mindestens 95 % und vorzugsweise mindestens 97 % 20-Hydroxyecdyson der Formel (I) aufweist.

15. 20-Hydroxyecdyson und dessen Derivate zur Verwendung nach einem der vorangehenden Ansprüche, **dadurch gekennzeichnet, dass** ein Derivat eine Verbindung der allgemeinen Formel (II) ist: 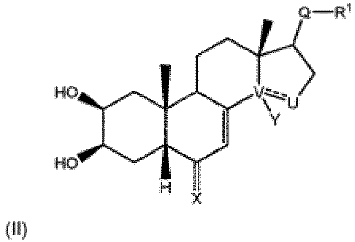 wobei:
**V-U** eine Kohlenstoff-Kohlenstoff-Einfachbindung ist und **Y** eine Hydroxyl- oder Wasserstoffgruppe ist, oder **V-U** eine ethylenische Bindung C=C ist;
**X** ein Sauerstoff ist,
**Q** eine Carbonylgruppe ist;
**R¹** ausgewählt ist aus: einer Gruppe (C₁-C₆)**W**(C₁-C₆); einer Gruppe (C₁-C₆)**W**(C₁-C₆)**W**(C₁-C₆); einer Gruppe (C₁-C₆)**W**(C₁-C₆)CO₂ (C₁-C₆); einer Gruppe (C₁-C₆)**A**, wobei **A** einen Heterozyklus darstellt, der eventuell durch eine Gruppe vom Typ OH, OMe, (C₁-C₆), N(C₁-C₆), CO₂(C₁-C₆) substituiert ist; einer Gruppe CH₂Br;
wobei **W** ein Heteroatom ist, das aus N, O und S ausgewählt ist.

16. 20-Hydroxyecdyson und dessen Derivate zur Verwendung nach vorangehendem Anspruch, **dadurch gekennzeichnet, dass** in der Formel (II):
**Y** eine Hydroxylgruppe ist;
**R¹** ausgewählt ist aus: einer Gruppe (C₁-C₆)**W**(C₁-C₆); einer Gruppe (C₁-C₆)**W**(C₁-C₆)**W**(C₁-C₆); einer Gruppe (C₁-C₆)**W**(C₁-C₆) CO₂ (C₁-C₆); einer Gruppe (C₁-C₆)**A**, wobei **A** einen Heterozyklus darstellt, der eventuell durch eine Gruppe vom Typ OH, OMe, (C₁-C₆), N(C₁-C₆), CO₂(C₁-C₆) substituiert ist ;
wobei **W** ein Heteroatom ist, das aus N, O und S ausgewählt ist.

17. 20-Hydroxyecdyson und dessen Derivate zur Verwendung nach einem der vorangehenden Ansprüche, **dadurch gekennzeichnet, dass** sie ausgewählt sind aus:
- **Nr. 1:** (2S,3R,5R,10R,13R,14S,17S)-2,3,14-trihydroxy-10,13-dimethyl-17-(2-morpholinoacetyl)-2,3,4,5,9,11,12,15,16,17-decahydro-1H-cyclopenta[a]phenanthren-6-one;
- **Nr. 2:** (2S,3R,5R,10R,13R,14S,17S)-2,3,14-trihydroxy-17-[2-(3-hydroxypyrrolidin-1-yl)acetyl]-10,13-dimethyl-2,3,4,5,9,11,12,15,16,17-decahydro-1H-cyclopenta[a]phenanthren-6-one;
- **Nr. 3:** (2S,3R,5R,10R,13R,14S,17S)-2,3,14-trihydroxy-17-[2-(4-hydroxy-1-piperidyl)acetyl]-10,13-dimethyl-2,3,4,5,9,11,12,15,16,17-decahydro-1H-cyclopenta[a]phenanthren-6-one;
- **Nr. 4:** (2S,3R,5R,10R,13R,14S,17S)-2,3,14-trihydroxy-17-[2-[4-(2-hydroxyethyl)-1-piperidyl]acetyl]-10,13-dimethyl-2,3,4,5,9,11,12,15,16,17-decahydro-1H-cyclopenta[a]phenanthren-6-one;
- **Nr. 5:** (2S,3R,5R,10R,13R,14S,17S)-17-[2-(3-dimethylaminopropyl(methyl)amino)acetyl]-2,3,14-trihydroxy-10,13-dimethyl-2,3,4,5,9,11,12,15,16,17-decahydro-1H-cyclopenta[a]phenanthren-6-one;
- **Nr. 6:** 2-[2-oxo-2-[(2S,3R,5R,10R,13R,14S,17S)-2,3,14-trihydroxy-10,13-dimethyl-6-oxo-2,3,4,5,9,11,12,15,16,17-decahydro-1H-cyclopenta[a]phenanthren-17-yl]ethyl]ethylsulfanylacetat;
- **Nr. 7:** (2S,3R,5R,10R,13R,14S,17S)-17-(2-ethylsulfanylacetyl)-2,3,14-trihydroxy-10,13-dimethyl-2,3,4,5,9,11,12,15,16,17-decahydro-1H-cyclopenta[a]phenanthren-6-one;
- **Nr. 8:** (2S,3R,5R,10R,13R,14S,17S)-2,3,14-trihydroxy-17-[2-(2-hydroxyethylsulfanyl)acetyl]-10,13-dimethyl-2,3,4,5,9,11,12,15,16,17-decahydro-1H-cyclopenta[a]phenanthren-6-one.

18. 20-Hydroxyecdyson und dessen Derivate zur Verwendung nach einem der vorangehenden Ansprüche, **dadurch gekennzeichnet, dass** das Derivat der Formel (III) entspricht:

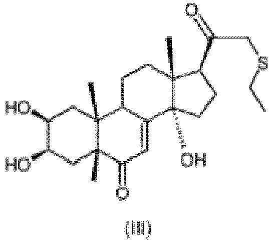

## Claims

1. 20-hydroxyecdysone and derivates thereof, for use in the treatment of a myopathy resulting from a genetic impairment including a mutation of the dystrophin gene.

2. 20-hydroxyecdysone and derivatives thereof, for use according to the preceding claim, for the treatment of illnesses resulting from an impairment of the muscular function caused by a genetic myopathy.

3. 20-hydroxyecdysone and derivatives thereof for use according to the preceding claim, **characterised in that** the muscular function is that of the skeletal striated muscle or of the myocardium.

4. 20-hydroxyecdysone and derivatives thereof for use according to the preceding claim, wherein the impairment of the muscular function is a hypertrophy of the myocardium.

5. 20-hydroxyecdysone and derivatives thereof for use according to any one of the preceding claims, **characterised in that** the muscular function is at least partly impaired by the progressive installation of a fibrosis.

6. 20-hydroxyecdysone and derivatives thereof, for use according to any one of the preceding claims, **characterised in that** the myopathy resulting from a genetic impairment is Duchenne muscular dystrophy (DMD) and/or Becker muscular dystrophy (BMD).

7. 20-hydroxyecdysone and derivatives thereof intended to be used according to any one of the preceding claims, **characterised in that** the mitochondrial respiration of the muscular cells is increased.

8. 20-hydroxyecdysone and derivatives thereof for use according to any one of the preceding claims, **characterised in that** the number of vessels per muscular fibre is increased.

9. 20-hydroxyecdysone and derivatives thereof for use according to any one of the preceding claims, **characterised in that** the differentiation of the myoblasts into myotubes is increased.

10. 20-hydroxyecdysone and derivatives thereof for use according to any one of the preceding claims, **characterised in that** the 20-hydroxyecdysone is a compound of formula (I):

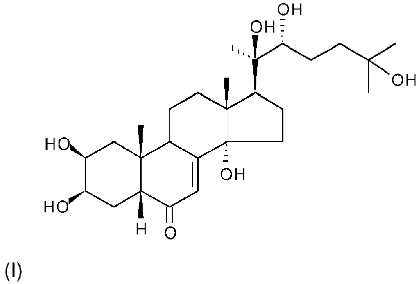

11. 20-hydroxyecdysone and derivatives thereof for use according to one of the preceding claims, **characterised in that** the 20-hydroxyecdysone is an extract of a plant or of a part of a plant, said plant being selected from plants containing at least 0.5% 20-hydroxyecdysone of formula (I), by dry weight of said plant, said extract including at least 95%, and preferably at least 97%, 20-hydroxyecdysone of formula (I).

12. 20-hydroxyecdysone and derivatives thereof for use according to the preceding claim, **characterised in that** the extract includes between 0 and 0.05%, by dry weight of the extract, impurities liable to affect the innocuousness, the availability or the efficacy of a pharmaceutical application of said extract.

13. 20-hydroxyecdysone and derivatives thereof for use according to any one of the preceding claims, **characterised in that** the plant is selected from *Stemmacantha carthamoides, Cyanotis arachnoidea* and *Cyanotis vaga.*

14. 20-hydroxyecdysone and derivatives thereof for use according to any one of the preceding claims, **characterised in that** the 20-hydroxyecdysone is an extract of roots of *Stemmacantha carthamoides* including at least 95%, and preferably at least 97%, 20-hydroxyecdysone of formula (I).

15. 20-hydroxyecdysone and derivatives thereof for use according to any one of the preceding claims, **characterised in that** a derivative is a compound of general formula (II): 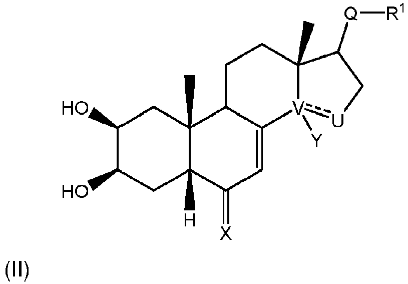 wherein:
**V-U** is a carbon-carbon single bond and **Y** is a hydroxyl group or a hydrogen atom, or **V-U** is a C=C ethylenic bond;
**X** is an oxygen atom,
**Q** is a carbonyl group;
**R¹** is selected from: a (C₁-C₆)**W**(C₁-C₆) group; a (C₁-C₆)W(C₁-C₆)**W**(C₁-C₆) group; a (C₁-C₆)**W**(C₁-C₆)CO₂(C₁-C₆) group; a (C₁-C₆)**A** group, **A** representing a heterocycle optionally substituted by a group of the OH, OMe, (C₁-C₆) or N(C₁-C₆), CO₂(C₁-C₆) type; a CH₂Br group;
**W** being a heteroatom selected from N, O and S.

16. 20-hydroxyecdysone and derivatives thereof for use according to the preceding claim, **characterised in that** in the formula (II):
**Y** is a hydroxyl group;
**R¹** is selected from: a (C₁-C₆)**W**(C₁-C₆) group; a (C₁-C₆)W(C₁-C₆)**W**(C₁-C₆) group; a (C₁-C₆)**W**(C₁-C₆) CO₂(C₁-C₆) group;
a (C₁-C₆)**A** group, **A** representing a heterocycle optionally substituted by a group of the OH, OMe, (C₁-C₆), N(C₁-C₆) or CO₂(C₁-C₆) type.
**W** being a heteroatom selected from N, O and S.

17. 20-hydroxyecdysone and derivatives thereof for use according to any one of the preceding claims, **characterised in that** they are selected from:
- **n°1 :** (2S,3R,5R,10R,13R,14S,17S)-2,3,14-trihydroxy-10,13-dimethyl-17-(2-morpholinoacetyl)-2,3,4,5, 9,11,12,15,16,17-decahydro-1H-cyclopenta[a]phenanthren-6-one;
- **n°2 :** (2S,3R,5R,10R,13R,14S,17S)-2,3,14-trihydroxy-17-[2-(3-hydroxypyrrolidin-1-yl)acetyl]-10,13-dimethyl-2,3,4,5,9,11,12,15,16,17-decahydro-1H-cyclopenta[a]phenanthren-6-one;
- **n°3 :** (2S,3R,5R,10R,13R,14S,17S)-2,3,14-trihydroxy-17-[2-(4-hydroxy-1-piperidyl)acetyl]-10,13-dimethyl-2,3,4,5,9,11,12,15,16,17-decahydro-1H-cyclopenta[a]phenanthren-6-one;
- **n°14:** (2S,3R,5R,10R,13R,14S,17S)-2,3,14-trihydroxy-17-[2-[4-(2-hydroxyethyl)-1-piperidyl)acetyl]10, 13-dimethyl-2,3,4,5,9,11,12,15,16,17-decahydro-1H-cyclopenta[a]phenanthren-6-one;
- **n°5:** (2S,3R,5R,10R,13R,14S,17S)-17-[2-(3-dimethylaminopropyl(methyl)amino)acetyl]-2,3,14-trihydroxy-10,13-dimethyl-2,3,4,5,9,11,12,15,16,17-decahydro-1**H**-cyclopenta[a]phenanthren-6-one;
- **n°6** : 2-[2-oxo-2-[(2S,3R,5R,10R,13R,14S,17S)-2,3,14-trihydroxy-10,13-dimethyl-6-oxo-2,3,4,5,9,11,12,15,16,17-decahydro-1H-cyclopenta[a]phenanthren-17-yl]ethyl]ethylsulfanylacetate;
- **n°7:** (2S,3R,5R,10R,13R,14S,17S)-17-(2-ethylsulfanylacetyl)-2,3,14-trihydroxy-10,13-dimethyl-2,3,4,5,9,11,12,15,16,17-decahydro-1H-cyclopenta[a]phenanthren-6-one;
- **n°8:** (2S,3R,5R,10R,13R,14S,17S)-2,3,14-trihydroxy-17-[2-(2-hydroxyethylsulfanyl)acetyl]-10,13-dimethyl-2,3,4,5,9,11,12,15,16,17-decähydrö-1H-cyclopenta[a]phenanthren-6-one.

18. 20-hydroxyecdysone and derivatives thereof for use according to any one of the preceding claims, **characterised in that** the derivative is of formula (III):
